(19)

Europäisches
Patentamt

European
Patent Office

Office européen
des brevets

(11)  **EP 1 930 444 A1**

(12)  **EUROPÄISCHE PATENTANMELDUNG**

(43) Veröffentlichungstag:
**11.06.2008  Patentblatt 2008/24**

(51) Int Cl.:
*C12Q 1/68* $^{(2006.01)}$         *G01N 33/68* $^{(2006.01)}$

(21) Anmeldenummer: **06025448.9**

(22) Anmeldetag: **08.12.2006**

(84) Benannte Vertragsstaaten:
**AT BE BG CH CY CZ DE DK EE ES FI FR GB GR
HU IE IS IT LI LT LU LV MC NL PL PT RO SE SI
SK TR**
Benannte Erstreckungsstaaten:
**AL BA HR MK RS**

(71) Anmelder:
  • **BioSpring Gesellschaft für Biotechnologie mbH
    60386 Frankfurt / Main (DE)**
  • **Johann Wolfgang Goethe-Universität Frankfurt
    am
    Main
    60325 Frankfurt am Main (DE)**

(72) Erfinder:
  • **Aygün, Hüseyin
    60322 Frankfurt am Main (DE)**
  • **Karas, Michael
    65795 Hattersheim (DE)**
  • **Bahr, Ute
    65795 Hattersheim (DE)**

(74) Vertreter: **Kalhammer, Georg et al
Lederer & Keller
Patentanwälte
Prinzregentenstrasse 16
80538 München (DE)**

(54)  **Massenspektrometrisches Verfahren an Proben enthaltend Nukleinsäuren**

(57)      Die Erfindung betrifft ein massenspektrometrisches Verfahren zum Nachweis und zur Quantifizierung von doppelsträngigen Nukleinsäuren, welche nicht kovalent miteinander assoziiert sind.

**EP 1 930 444 A1**

SEQUENCE LISTING

```
<110>  BioSpring Gesellschaft für Biotechnologie mbH
       Johann Wolfgang Goethe-Universität Frankfurt am Main

<120>  Massenspektrometrisches Verfahren an Proben enthaltend
       Nukleinsäuren

<130>  MALDI

<160>  2

<170>  PatentIn version 3.3

<210>  1
<211>  19
<212>  RNA
<213>  artificial

<220>
<223>  Probensequenz (RNA)


<220>
<221>  modified_base
<222>  (1)..(1)
<223>  gm

<220>
<221>  modified_base
<222>  (3)..(3)
<223>  cm

<220>
<221>  modified_base
<222>  (5)..(5)
<223>  2' O-methyl adenosin

<220>
<221>  modified_base
<222>  (7)..(7)
<223>  2' O-methyl adenosin

<220>
<221>  modified_base
<222>  (9)..(9)
<223>  um

<220>
<221>  modified_base
<222>  (11)..(11)
<223>  um

<220>
<221>  modified_base
<222>  (13)..(13)
<223>  cm

<220>
<221>  modified_base
<222>  (15)..(15)
<223>  2' O-methyl adenosin

<220>
<221>  modified_base
<222>  (17)..(17)
<223>  2' O-methyl adenosin

<220>
<221>  modified_base
```

```
<222>   (19)..(19)
<223>   um

<400>   1
ggcgauauuc ugcuacagu                                                    19


<210>   2
<211>   19
<212>   RNA
<213>   artificial

<220>
<223>   Probensequenz (RNA)

<400>   2
acuguagcag aauaucgcc                                                    19
```

**Beschreibung**

Nukleinsäuren sind aus einzelnen Bausteinen, den Nukleotiden, aufgebaute Makromoleküle. Alternierende Monosaccharide und Phosphorsäurereste bilden hierbei das Grundgerüst der Nukleinsäuren, wobei an jedem Monosaccharid eine Nukleinbase hängt. Wichtige Vertreter der Nukleinsäuren sind die Desoxyribonukleinsäure (DNA), Ribonukleinsäure (RNA), aber auch Derivate und Modifikationen dieser, wie die "locked nucleic acid" (LNA), die eine modifizierte RNA darstellt. Des Weiteren werden oft auch DNA- und RNA-ähnliche organische Polymere, wie z.B. Peptid-Nukleinsäure (PNA) vom Oberbegriff der Nukleinsäure umfasst.

Eine wesentliche Eigenschaft der Nukleinsäuren liegt darin, dass sie nicht nur als Einzelstränge des Polymers aus Nukleotiden oder Derivaten dieser vorliegen kann, sondern auch in Form von Doppelsträngen oder komplexeren Formen (z.B. als Tripelhelix) vorliegt. Eine Besonderheit hierbei ist, dass doppelsträngige bzw. komplexere Strukturen nicht aus beliebigen Einzelsträngen der Nukleinsäuren gebildet werden, sondern die Bildung in Abhängigkeit der Basensequenz abläuft. So ist ein bestimmter Anteil an Komplementarität in der Basenabfolge zweier einzelner Nukleinsäurestränge nötig, um eine doppelsträngige Struktur bilden zu können. Liegt ausreichende Komplementarität vor, bilden sich solche Strukturen, wie eine Doppelstrangstruktur, spontan nach Zusammenbringen der Nukleinsäure-Einzelstränge unter physiologischen Bedingungen (Salzkonzentration, pH und Temperatur). Stabilisiert werden Doppelstränge durch hydrophobe Wechselwirkungen innerhalb der Basenstapelung (stacking interactions) und durch intermolekulare Wasserstoffbrücken der jeweils komplementären Basen der Einzelstränge.

Im Stand der Technik werden doppelsträngige Nukleinsäuren gewonnen, indem man entweder die komplementären Einzelstränge getrennt synthetisiert und anschließend unter physiologischen Bedingungen zum Doppelstrang kombiniert, oder den Doppelstrang hintereinander in einer sog. "Tandem-"Synthese des Einzelstranges synthetisiert. Hierbei wird nach der Einzelstrangsynthese nach chemischer Aufarbeitung ein bei der Synthese eingebrachter Linker hydrolysiert und die entstehenden Einzelstränge, welche komplementäre Basenabfolgen aufweisen, werden anschließend unter physiologischen Bedingungen hybridisiert.

In neuester Zeit stoßen gerade doppelsträngige RNA-Moleküle auf sehr großes Interesse. Die als RNAi (RNA-Interferenz oder RNA interference) bezeichnete Technologie, bei welcher kurze doppelsträngige RNA-Sequenzen zum Einsatz kommen (sog. siRNA's), nutzt eine spezielle Eigenschaft höherer Zellen aus, um die intrazelluläre Proteinexpression auf mRNA-Ebene mittels solcher Doppelstränge und weiterer zelleigener Komponenten zu regulieren. Elbashir und Tuschl gelang 2001 erstmals der Nachweis, dass sich ein solcher Prozess auch mit Hilfe von kurzen doppelsträngigen RNA-Molekülen (siRNA's) induzieren lässt (Elbashir SM, Lendeckel W, and Tuschl T, Genes Dev, 2001, 15(2), 188 - 200; Elbashir SM, Harborth J, Lendeckel W, Yalcin A, Weber K, Tuschl T, Nature, 2001, 411, 494 - 498; Caplen NJ, Parrish S, Imani F, Fire A, and Morgan RA, Proc. Natl. Acad. Sci. USA, 2001, 98, 9746 - 9747). In der Regel handelt es sich hierbei um chemisch hergestellte Doppelstränge, mit einer Länge von 19 - 21 Basenpaaren und einem Dinukleotid als 3'-Überhang (zumeist dTdT).

Für die erfolgreiche Anwendung doppelsträngiger Strukturen in Forschung und Medizin ist es besonders wichtig, neben der Reinheit der Einzelstränge auch die Vollständigkeit der Hybridisierung als Doppelstrang zu gewährleisten. Ein Überschuss an Einzelsträngen kann in der Anwendung zu zahlreichen Nebenwirkungen führen und sollte daher möglichst vermieden werden. Wichtig ist dabei die Fähigkeit von Zellen, über sog. TLR-Rezeptoren an Membranoberflächen solche einzelsträngigen Nukleinsäuren zu erkennen und darauf mittels einer Immunantwort (z.B. durch Ausschüttung von Interferonen) zu reagieren. Diese Immunantwort ist jedoch insbesondere bei medizinischen Verfahren unerwünscht und sollte vermieden werden. Für RNA-Einzelstränge sind es vor allem die Rezeptoren TLR 7 und TLR 8, die in der Lage sind, eine solche Immunantwort auszulösen (Heil F, Hemmi H, Hochrein H, Ampenberger F, Kirschning C, Akira S, Lipford G, Wagner H, Bauer S, Science, 2004, 303, 1526 - 1529). Daher sollten Anwendungen, in welcher doppelsträngige Nukleinsäuren als aktive Substanz wirken, im Hinblick auf das Vorhandensein von überschüssigen Einzelsträngen gut charakterisiert werden.

In der Regel werden für die Herstellung eines Doppelstranges gleiche Anteile der beiden komplementären Einzelstränge benötigt. Um dies quantitativ erreichen zu können werden Nukleinsäuren, z.B. synthetische Oligonukleotide, im Stand der Technik mittels UV-VIS Spektroskopie quantifiziert. Hierbei werden die Extinktionskoeffizienten einer Lösung, die die Nukleinsäure enthält, gemessen und der Nukleotidgehalt über das Lambert-Beersche Gesetz berechnet. Der Extinktionskoeffizient einer Nukleinsäure setzt sich näherungsweise additiv aus den Extinktionskoeffizienten der Einzelbasen eines Stranges zusammen. Ein Fehler dieser additiven Abschätzung des Extinktionskoeffizienten der Nukleinsäure aus den Einzelbasen entsteht jedoch durch nachbarschaftliche Wechselwirkungen der Einzelbasen, die die effektive Absorption der Einzelbasen verändert, wodurch sich der Extinktionskoeffizient des Gesamtstrangs nicht mehr exakt berechnen lässt. Hinzu kommt, dass die Nukleinsäuren selbst unter den Messbedingungen intramolekulare Strukturen bilden können, was die Extinktionskoeffizienten der Einzelbasen über den sog. hyperchromen Effekt weiter verändert, und somit den berechneten Wert des Oligonukleotidgehalts weiter verfälscht. Dies hat zur Folge, dass es zu Fehlern bei der Bestimmung der Einzelstrangkonzentration kommt. Durch die Fehler bei der Bestimmung der Einzelstrangkonzentration einer Probe wird folglich bei der Herstellung eines Doppelstrangs ein Produkt erhalten, das neben den gewünschten Doppelsträngen einen Überschuss an einem Einzelstrang enthält. Solche Proben sind jedoch unvorteilhaft, da sie wie oben ausgeführt insbesondere bei medizinischen Verfahren unerwünschte Nebenwirkungen auslösen.

Die erhaltenen Proben, die neben dem Anteil an Doppelstrang einen weiteren Anteil an Einzelstrang aufweisen können, müssen insbesondere durch zwei Messwerte charakterisiert werden. Zum einen handelt es sich um die qualitative Erfassung des Einzelstrangüberschusses (Einzelstrang A oder komplementärer Einzelstrang B), zum anderen um das Verhältnis von Einzel- zu Doppelstrang in der Probe.

Der Einzelstranganteil wird im Stand der Technik über Ionentauscher- oder Umkehrphasen-HPLC bestimmt. Dieses Verfahren ist jedoch aufwendig, zeitintensiv und setzt zudem eine ständige Optimierung der Laufbedingungen voraus. Um dieses Verfahren außerdem quantitativ anwenden zu können, werden von jedem Einzelstrang reine Proben als Kalibrierungsstandards benötigt. Die Detektionsgrenze liegt hierbei für den Einzelstranganteil im Bereich um 5%. Dies lässt sich u.a. damit erklären, dass die Bestandteile (Strang A, B, oder Doppelstrang) häufig ähnliche Retentionszeiten in der Chromatographie aufweisen. Eine Auswertung wird somit durch das "Ineinanderlaufen" beider Komponenten wesentlich erschwert und ungenau. Zudem lassen sich über HPLC keine direkten Aussagen über den im Überschuss befindlichen Einzelstrang machen (Strang A oder Strang B). Weisen beide Stränge keinen oder einen zu geringen Retensionsunterschied in der verwendeten HPLC-Methode auf, muss die Bestimmung des Einzelstrangüberschusses über eine weitere Methode erfolgen.

Ein weiteres Verfahren, das Verhältnis an Einzelstrang zu Doppelstrang zu bestimmen, ist die Polyacrylamidgelelektrophorese (PAGE). Hierbei wird der Umstand ausgenutzt, dass im elektrischen Feld die Wanderungseigenschaften von Doppelsträngen und Einzelsträngen, durch die Ladungsverhältnisse bedingt, deutlich unterschiedlich sind. Auch bei dieser Methode dominieren zahlreiche Einschränkungen. Die genaue Quantifizierung des Überschussanteils, auch hierfür werden Eichsubstanzen benötigt, sowie die Charakterisierung des im Überschuss vorliegenden Einzelstranges erweisen sich als fehlerbehaftet und wenig vorteilhaft, insbesondere wenn beide Stränge die gleiche Länge aufweisen und somit ähnliches Laufverhalten im Gel aufweisen.

Ein weiteres bekanntes Verfahren zur Untersuchung von Biomolekülen ist die Massenspektrometrie (MS). Sie stellt unter anderem ein einfaches und sehr effizientes Messverfahren zur Bestimmung der molekularen Masse von Biomolekülen dar (W. D. Lehmann: Massenspektrometrie in der Biochemie, Spektrum Akad. Verlag, Heidelberg 1996) und wird hauptsächlich zur Analyse von Proteinen, aber auch Nukleinsäuren eingesetzt.

Als analytisches Verfahren kommt die Massenspektrometrie häufig zum Einsatz, um die Qualität synthetisch hergestellter Nukleinsäuren nach der Herstellung zu überprüfen. Bekannt ist z.B. ein Verfahren, dass als LC-MS bezeichnet wird. Hierbei wird die Flüssigchromatographie mit der Massenspektrometrie gekoppelt. Die Flüssigchromatographie dient zur Auftrennung und die Massenspektrometrie zur Identifikation und/oder Quantifizierung der Substanzen. In der Regel werden noch weitere Detektoren zugeschaltet, wie z.B. UV-ELS- oder Leitfähigkeitsdetektoren. Hierdurch können z.B. qualitative und quantitative Aussagen über Verunreinigungen in der zu untersuchenden Oligonukleotidprobe festgestellt werden. Am häufigsten findet jedoch eine als MALDI-MS (matrix assisted laser desorption ionisation mass spectrometry) Verfahren bezeichnete Technik Anwendung (M. Karas, F. Hillenkamp, Anal. Chem. 1988, 60, 2299; M. Karas, U. Bahr, F. Hillenkamp, Int. J. Mass Spectrom. Ion Proc. 1989, 92, 231; M. Karas, U. Bahr, A. Ingendoh, F. Hillenkamp, Angew. Chemie 1989, 101, 805; F. Hillenkamp, M. Karas, Methods Enzymol. 1990, 193, 280). Diese Technik eignet sich besonders zur schnellen und qualitativen Untersuchung von Oligonukleotiden (Bonk T, Humeny A, The Neuroscientist, 2001, 7 (1), 6 - 12; Distler AM, Allison J, Anal Chem., 2001, 73 (20), 5000 - 5003). Im Stand der Technik sind jedoch nur wenige erfolgreiche Anwendungen dieser Methode übermittelt, bei denen es gelungen ist, intakte Biomoleküle zu detektieren, die nicht über kovalente Wechselwirkungen stabilisiert werden, wie beispielsweise Nukleinsäuredoppelstränge.

Ursachen für die Schwierigkeiten, nicht-kovalent miteinander assoziierte Doppelstränge mit MALDI-MS nachzuweisen, sind die im Stand der Technik verwendete Probenmatrix, die einen sauren pH-Wert von ca. 3 aufweist, sowie die Verwendung von organischen Lösungsmitteln. Beides führt zur Destabilisierung von Nukleinsäuredoppelsträngen.

Inwieweit die zur Desorption nötige Laserintensität (UV-Laser) zur Destabilisierung beiträgt, ist bisher nicht bekannt.

Zahlreiche Variationen wurden entwickelt, um den Nachweis von nicht-kovalent assoziierten Einzelsträngen in der MALDI-Massenspektrometrie zu ermöglichen.

Lecci und Pannell (J. Am. Soc. Mass Spectrom., 1995, 6, 972 - 975) beschreiben die Verwendung von 6-Aza-2-thiothymin anstelle von 3-Hydroxypicolinsäure als Probenmatrix, um doppelsträngige DNA über MALDI-MS nachzuweisen. Jedoch konnte auch durch die Veränderung der Probenmatrix nicht verhindert werden, dass signifikante Mengen von Einzelsträngen, verursacht durch die Dissoziation des Doppelstranges, während der Desorption gemessen wurden. Eine Aussage über das Verhältnis von überschüssigem Einzelstrang zum Doppelstrang der Probe kann somit nicht vorgenommen werden.

Little et al. (Int. J. Mass Spectrom. Ion Processes, 169/170, 1997, 323 - 330) beschreibt die Untersuchung von DNA mittels MALDI-MS, bei der die Probenmatrix zusätzlich abgekühlt wurde, um die Dissoziation der Doppelstränge zu verhindern. Die Abkühlung stellt jedoch einen weiteren aufwendigen Prozessschritt dar, der unvorteilhaft ist, und ebenfalls nicht verhindern konnte, dass signifikante Mengen an Einzelsträngen bei der Messung nachgewiesen wurden. Somit ist auch durch dieses Verfahren keine Möglichkeit gegeben, das Verhältnis von überschüssigem Einzelstrang zum Doppelstrang der Probe zu bestimmen.

Eine weitere Methode, mittels Massenspektrometrie doppelsträngige Nukleinsäureproben zu untersuchen, wurde von Kirpekar et al. beschrieben (Kirpekar F, Berkenkamp S, Hillenkamp F, Anal. Chem., 1999, 71 (13), 2334 - 2339). Neben UV-Lasern und 6-Aza-2-thiothymin als Probenmatrix wurde hier statt eines handelsüblichen Stickstofflasers ein IR-Laser zur Probendesorption verwendet. Das Probenmaterial bestand aus sehr langen Oligonukleotid-Doppelsträngen (70mer und 80mer), von denen Doppelstränge nachgewiesen, aber nicht quantifiziert wurden. Auch diese Methode kann somit keine Aussage über das Verhältnis von überschüssigem Einzelstrang zum Doppelstrang liefern.

Es besteht somit Bedarf an einem schnellen, unkomplizierten Verfahren zur Bestimmung von Eigenschaftsparametern einer Probe enthaltend mindestens ein Biomolekül, vorzugsweise mindestens eine Nukleinsäure, wodurch die Eigenschaftsparameter, bei nukleinsäurehaltigen Proben insbesondere das Verhältnis von Einzelstrang zu Doppelstrang der Nukleinsäure der Probe und/oder der Überschuss an einem Einzelstrang an Nukleinsäure in der Probe, bestimmt werden können.

Überraschenderweise wurde gefunden, dass bestimmte Eigenschaftsparameter einer Probe, die mindestens ein Biomolekül, vorzugsweise mindestens eine Nukleinsäure enthält, durch Ausführung des nachfolgend beschriebenen Verfahrens quantitativ bestimmt werden können.

Die Erfindung betrifft ein Verfahren zur Bestimmung wenigstens eines Eigenschaftsparameters einer Probe, die wenigstens ein Biomolekül enthält, wobei das Verfahren die folgenden Schritte umfasst:

(a) Zugabe eines Standards zu der Probe;
(b) Erstellen eines Massenspektrums der den Standard enthaltenden Probe unter nativen Bedingungen;
(c) Erstellen eines Massenspektrums der den Standard enthaltenden Probe unter denaturierenden Bedingungen;
(d) Vergleichen der Peakhöhe oder Peakfläche von wenigstens einem dem Biomolekül zuzuordnenden Peak in dem Massenspektrum aus Schritt (b) mit der Peakhöhe bzw. Peakfläche des korrespondierenden Peaks in dem Massenspektrum aus Schritt (c).

Als Biomolekül wird im Rahmen der vorliegenden Erfindung eine chemische Verbindung verstanden, die in der Natur in lebenden Organismen auftritt oder von diesen hergestellt wird oder werden kann. Biomoleküle bestehen hauptsächlich aus Kohlenstoff und Wasserstoff, zusammen mit Stickstoff, Sauerstoff, Phosphor, Schwefel und weiteren selteneren Elementen. Gegebenenfalls bestehen die Biomoleküle im Rahmen der vorliegenden Erfindung aus zwei nicht kovalent gebundenen Einheiten, wie z.B. homo- oder heterodimere Proteinkomplexe oder doppelsträngige DNA, wobei letztere bevorzugt ist.

In einer bevorzugten Ausführungsform des erfindungsgemäßen Verfahrens umfasst das Biomolekül mindestens eine Nukleinsäure, insbesondere ein oder mehrere Oligomoleküle, z.B. ein Gemisch aus einzelsträngigen und/ oder doppelsträngigen Nukleinsäuren.

Im Rahmen der vorliegenden Erfindung wird unter "Doppelstranganteil" in der Probe das Verhältnis der Mengen (Stoffmengen) des Teils einer Nukleinsäure, die als Doppelstrang vorliegt zu dem Teil einer Nukleinsäure, der als Einzelstrang vorliegt, verstanden. Bei den Teilen, die als Doppelstrang bzw. Einzelstrang vorliegen, handelt es sich jeweils um zwei komplementäre Einzelstränge, die zusammen einen Doppelstrang bilden können. Es kann somit für jeden der komplementären Einzelstränge, die vorzugsweise nicht identisch sind, ein Doppelstranganteil bzw. ein Verhältnis des jeweiligen Einzelstrangs zu dem im Doppelstrang vorliegenden Teil in der Probe bestimmt werden.

Als die in der Probe enthaltenen Nukleinsäuren können DNA, RNA, nicht natürliche Derivate hieraus wie LNA und vorzugsweise natürliche oder synthetisch hergestellte Nukleinsäuren, sog. Oligonukleotide, verwendet werden.

Als Eigenschaftsparameter können durch das erfindungsgemäße Verfahren insbesondere der Doppelstranganteil der Probe, das Verhältnis von Einzelsträngen zu Doppelsträngen in der Probe, der Überschuss an Einzelstrang in der Probe, die Wechselwirkungskräfte zwischen verschiedenen Nukleinsäuren der Probe oder die Wechselwirkung der in der Probe vorhandenen Nukleinsäure mit anderen nicht-Nukleinsäure-haltigen Inhaltsstoffen der Probe bestimmt werden.

Im Weiteren wird das erfindungsgemäße Verfahren beispielhaft für die Messung von Nukleinsäuren, insbesondere doppelsträngige und einzelsträngige Nukleinsäuren beschrieben. Es kann jedoch entsprechend auf weitere Biomoleküle, wie z.B. Proteine, die entsprechend in Komplexen bestehend aus zwei nicht kovalent gebundenen Einheiten, was dem Doppelstrang entsprechen würde, und in entsprechenden Monomeren, was dem Einzelstrang entsprechen würde, übertragen werden.

Unter "Vergleichen" der Peakhöhen oder Peakflächen einzelner Peaks wird das ins Verhältnissetzen bzw. das Umrechnen nach algebraischen Vorschriften, wie z.B. im Rahmen dieser Anmeldung beschrieben, der numerischen Werte, die für die entsprechenden Peaks aus den Spektren bestimmt werden können, z.B. count, area, usw. verstanden. Das Quantifizieren von Peaks aus Spektren ist im Stand der Technik bekannt.

Als "korrespondierend" werden im Rahmen der vorliegenden Anmeldung Peaks bezeichnet, die in mehreren Messungen auftreten und von der gleichen Substanz stammen. Hierbei werden z.B. als korrespondierende Peaks diese bezeichnet, welche von einem bestimmten Einzelstrang stammen, der unter nativen Bedingungen im Spektrum nur in der Höhe zu sehen ist, in der er nicht im Doppelstrang gebunden ist, und unter denaturierenden Bedingungen ein stärkeres Signal gibt, da praktisch kein Doppelstrang mehr vorliegt. Vorzugsweise werden im Rahmen der vorliegenden Erfindung als korrespondierende Peaks die jeweils einem bestimmten Einzelstrang zuzuordnenden Peakpaare von zwei Messungen, von denen eine unter nativen und eine unter denaturierenden Bedingungen aufgenommen ist, verstanden.

Unter "Normalisierung" oder "Normalisieren" wird im Rahmen der vorliegenden Erfindung das Multiplizieren der Peakhöhen/-flächen eines Spektrums bezüglich eines anderen mit einem Faktor verstanden, der so bestimmt wird, dass eine Substanz, vorzugsweise der Standard, die bei beiden Messungen konstant gehalten wurde, anschließend in beiden Spektren die selbe Peakhöhe oder -fläche aufweist. Vorzugsweise werden bei dem erfindungsgemäßen Verfahren alle Messungen bezüglich einer Messung, insbesondere bezüglich des in den Proben aller Messungen konstant gehaltenen Standards, normalisiert.

Das Massenspektrum einer Probe wird durch Messung der Probe in einem Massenspektrometer erhalten. Als im erfindungsgemäßen Verfahren einsetzbarer Massenspektrometer ist ein MALDI-basiertes Massenspektrometer bevorzugt. Im MALDI-Massenspektrometer wird die Probe in Form einer sog. Matrix in das Gerät eingebracht. Die MALDI-Massenspektrometrie beruht vor allem auf der Kokristallisation von Matrix und Analyt mit einem 100 bis 100.000-fachen molekularen Überschuss an Matrix. Als Matrixsubstanzen werden normalerweise kleine organische Moleküle gewählt, die bei der verwendeten Laserwellenlänge (z.B. Stickstoff-Laser bei einer Wellenlänge von 337nm) stark absorbieren. Mit kurzen hochenergetischen Laserimpulsen von einigen Nanosekunden Pulsdauer folgt die Anregung, die nach Relaxation im Kristallgitter zu explosionsartigen Teilchenablösungen an der Oberfläche des Matrixkristalls führt. Durch die Verbindung mit der Matrix wird eine Fragmentierung von massenreichen Molekülen verhindert. Der Ionisierungsmechanismus bei der MALDI-Massenspektrometrie ist noch nicht vollständig verstanden.

Überraschenderweise wurde festgestellt, dass bei besonders hohen Konzentrationen an Matrixpuffer (Diammoniumhydrogencitrat, DAHC) und bei optimaler Probenkonzentration, sowie Vermeidung von organischen Lösungsmitteln der Doppelstranganteil einer Nukleinsäure enthaltenden Probe, selbst in einer handelsüblichen Probenmatrix wie 6-Aza-2-Thiotymin (ATT) während der Desorption mittels UV-Laser stabil erhalten bleibt. Somit wird eine quantitative Bestimmung des Doppelstranganteils in der Probe möglich.

Der vorliegenden Erfindung liegt unter anderem das Problem zugrunde, dass zur Quantifizierung des Gehalts an Doppelstrang einer Probe, z.B. bestehend aus zwei Oligonukleotiden, die Signalintensitäten der Doppel- und der Einzelstränge im MALDI-Massenspektrum nicht direkt miteinander verglichen werden können, da die erhaltenen Intensitäten sowohl von der Masse der detektierten Nukleinsäure als auch von der Basenzusammensetzung selbst abhängen.

Ein Problem bei der quantitativen Bestimmung des Verhältnisses von Einzelstrang zu Doppelstrang und Bestimmung des Überschussanteils an einem Einzelstrang gegenüber dem komplementären Einzelstrang liegt in der Variation der absoluten Werte der Peakhöhen/Peakflächen einer massenspektrometrischen Messung zur nächsten. Diese Probleme wurden durch das Einführen einer Referenzsubstanz, welche sich in der Probe als interner Standard befindet, gelöst. Somit war eine Quantifizierung des Verhältnisses von Einzelstrang zu Doppelstrang und des Überschussanteils an einem Einzelstrang erstmals mit Hilfe der MALDI-MS-Technik möglich.

Bei dem erfindungsgemäßen Verfahren werden mindestens zwei Messungen durchgeführt, eine unter nativen Bedingungen, bei denen der Doppelstrang stabilisiert ist, und eine unter denaturierenden, den Doppelstrang destabilisierenden, Bedingungen. Ein Intensitätsvergleich der Peaks der korrespondierenden Einzelstränge unter Berücksichtigung des internen Standards liefert hierbei eine verlässliche Aussage über den Einzelstranganteil der Probe. Insbesondere wird jeweils bei zwei Messungen, unter nativen und unter denaturierenden Bedingungen, der gelösten Probe ein interner Standard zugesetzt und die Einzelstrangintensität ($I_{ss}$) relativ zur Intensität des internen Standards ($I_{ss}$) bestimmt. Aus den Einzelstrangintensitäten lassen sich Einzelstrang- und Doppelstranganteil berechnen. Des Weiteren lässt sich simultan bestimmen, um welchen Einzelstrang es sich im Überschuss handelt, sofern sich die Massen der beiden Stränge unterscheiden ($M_{Strang\ A} \neq M_{Strang\ B}$).

Zur Messung unter "nativen" Bedingungen, d.h. unter optimierten MALDI-Massenspektrometriebedingungen, bei denen insbesondere auch die Doppelstränge erhalten bleiben und als solche quantitativ bestimmt werden können, wird zunächst eine wässrige Lösung hergestellt, die aus der Oligonukleotidprobe sowie einem zugesetzten Standard besteht. Der Standard, der z.B. ein weiteres Oligonukleotid umfasst, darf nicht unter den experimentellen Bedingungen, insbesondere nicht unter "nativen" und "denaturierenden" Bedingungen, mit der Probe wechselwirken. Der zugesetzte Standard fungiert hierbei als interner Standard.

Eine weitere Messung erfolgt anschließend unter "denaturierenden" Bedingungen. Hierfür wird der wässrigen Lösung der Probe, die aus der Nukleinsäure besteht, neben dem zugesetzten Standard ein denaturierendes Agens, z.B. Ameisensäure hinzugesetzt. Das denaturierende Agens wird so gewichtet und in solchen Mengen zugesetzt (vorzugsweise 10 - 30 Gew.-%, insbesondere 15 - 25 Gew.-%, z.B. 20 Gew.-% der Probe), dass der Doppelstrang vollständig in Einzelstränge zerfällt. Die zuvor unter "nativen" Bedingungen durchgeführte Messung wird somit unter identischen Bedingungen unter lediglicher Zugabe von denaturierendem Agens wiederholt.

Da die Konzentration des Standards in beiden Messungen identisch ist, wird ein Vergleich der einen Messung unter "nativen" Bedingungen und der anderen Messung unter "denaturierenden" Bedingungen unter Berücksichtigung des Verhältnisses der Intensitäten der Signale für den Standard ermöglicht. Hierfür wird ein Korrekturfaktor y eingeführt, der dem Verhältnis der Intensitäten des Standards beider Messungen wiedergibt.

$$y = \frac{I_{Standard\ 1}}{I_{Standard\ 2}} \qquad (1)$$

Aus der Messung unter "nativen" Bedingungen ist die Intensität des Einzelstrangs, der nicht im Doppelstrang gebunden ist, bekannt ($I_{SSNativ}$). Aus der Messung unter "denaturierenden" Bedingungen ist die Intensität und damit die Menge des insgesamt in der Probe enthaltenen Einzelstranges bekannt ($I_{SSDenat}$). Unter Berücksichtigung des Verhältnisfaktors y zwischen den beiden unterschiedlichen Messungen kann hieraus der Anteil des Einzelstrangs bestimmt werden, zu dem er unter "nativen" Bedingungen vorliegt ($1_{SSNativ}$ (%)). Da dieser Anteil sich mit dem Anteil an Einzelstrang, der im Doppelstrang gebunden vorliegt, zu 100% ergänzt, kann hieraus der Anteil an Einzelstrang bestimmt werden, der im Doppelstrang gebunden vorliegt ($I_{DS}$ (%)).

$$I_{SSNat}\ (\%) = y \cdot \frac{I_{SSNat}}{I_{SSDenat}} \cdot 100 \qquad (2)$$

$$I_{DS}\ (\%) = 100 - I_{SSNat}\ (\%) \qquad (3)$$

Zur Quantifizierung können neben den Peakflächen auch die Peakhöhen verwendet werden, vorausgesetzt Standard und Einzelstränge liegen in einem ähnlichen Massenbereich, sodass die Auflösung (Peakbreite) für beide gleich ist.

Als Standardsubstanz eignen sich alle einzelsträngigen Oligonukleotide, die unter den experimentellen Bedingungen keine Wechselwirkung mit den Analytensubstanzen zeigen und sich nicht unter Einfluss des denaturierenden Agens verändern. Durch den Einsatz von Oligonukleotiden kann auch leicht der Massenbereich des Standards auf die Einzelstränge abgestimmt werden, was wie oben ausgeführt zu einer vergleichbaren Auflösung führt. Vorteilhaft ist eine Differenz von 1-2 Nukleotidbausteinen zwischen der Länge der Oligonukleotide und der Länge der zu untersuchenden Einzelstränge.

Eine derartige Analyse der erhaltenen Spektren setzt eine Linearität der massenspektrometrischen Messung über zwei Größenordnungen an Konzentration voraus, d.h. die Konzentrationen der Einzelstränge liegen zwischen 1 bis 100 Prozent des Messbereichs des Massenspektrometers. Des Weiteren muss beachtet werden, dass bei der Messung unter "denaturierenden" Bedingungen im Vergleich zur Messung bei "nativen" Bedingungen das Verhältnis der Ionenintensität von Standard und Analyt nicht beeinflusst werden darf, um eine Verfälschung der Ergebnisse der beiden Messungen zu vermeiden.

Neben der oben angegebenen Bestimmung des Anteils, in dem ein Einzelstrang im Doppelstrang gebunden ist, kann aus den beiden Messungen bei "nativen" und "denaturierenden" Bedingungen ein eventuell vorhandener Überschuss des einen der beiden Einzelstränge (Strang 1 oder 2) bestimmt werden, die den Doppelstrang bilden. Der direkte Unterschied der Signalintensitäten der beiden Stränge innerhalb eines Spektrums kann hierfür nicht direkt herangezogen werden, da aufgrund unterschiedlicher Ionisierungs- und Nachweiswahrscheinlichkeiten der beiden Moleküle der Überschuss nicht direkt, z.B. durch Subtraktion der Intensitäten, erhalten werden kann. Die unterschiedliche Ionisierungs- und Nachweiswahrscheinlichkeit eines Moleküls wird mittels eines unbekannten Responsefaktors z berücksichtigt. Da der Responsefaktor z eines Strangs bezüglich des anderen zwischen zwei Messungen, z.B. unter "nativen" und "denaturierenden" Bedingungen, gleich bleibt, kann dieser unbekannte Faktor z über das Verhältnis der Intensitäten zwischen den Messungen eliminiert werden. Auf dieses Weise kann der Überschuss eines Einzelstrangs gegenüber dem anderen quantitativ bestimmt werden.

$$I_{1D} = z \, (I_{2D} + I_Y)$$

$$I_{1N} = z(I_{2N} + I_Y)$$

$$\frac{I_{1D}}{I_{2D} + I_Y} = \frac{I_{1N}}{I_{2N} + I_Y}$$

$$I_Y = \frac{I_{1D} \cdot I_{2N} - I_{1N} \cdot I_{2D}}{I_{1N} - I_{1D}}$$

wobei $I_Y$ den Überschuss eines Einzelstrangs, z den unbekannten Response-Faktor, $I_{1D}$ und $I_{2D}$ die normierten Intensitäten der Einzelstränge 1 und 2 unter "denaturierenden" und $I_{1N}$ und $I_{2N}$ die Intensitäten der Einzelstränge 1 und 2 unter "nativen" Bedingungen darstellt.

Im Falle eines Überschusses an Einzelstrang 1 ist $I_Y$ positiv, im Falle eines Überschusses von Einzelstrang 2 ist $I_Y$ negativ, liegt kein Überschuss eines Einzelstrangs vor ist $I_Y = 0$.

Für Überschuss an Einzelstrang 1 kann anschließend gemäß Gleichung (4a) der relative Überschuss an Einzelstrang 1 ($I_{Y1}$ (%)) bzw. für Überschuss an Einzelstrang 2 der relative Überschuss an Einzelstrang 2 ($I_{Y2}$ (%)) gemäß Gleichung (4b) berechnet werden.

$$I_{Y1} \, (\%) = \frac{I_Y}{I_{1D} - I_Y} \cdot 100 \qquad (4a)$$

$$I_{Y2} \, (\%) = \frac{|I_Y|}{I_{2D} - |I_Y|} \cdot 100 \qquad (4b)$$

Das vorgestellte erfindungsgemäße Verfahren der Doppelstrangquantifizierung hat insbesondere den Vorteil, dass die Peakverhältnisse in einer einzigen Lösung bestimmt werden. Durch das Ins-Verhältnis-setzen der Intensitäten der Peaks zweier Messungen werden Schwankungen der Messparameter eliminiert und somit eine hohe Genauigkeit der zu bestimmenden Werte gewährleistet.

Das erfindungsgemäße Verfahren hat weiterhin die Vorteile, dass die Messung aufgrund der Verwendung eines MALDI-Massenspektrometers sehr schnell ist, da die Methode automatisierbar und der Probendurchsatz sehr hoch ist. So werden typischerweise zur Aufnahme der beiden Messungen bei "nativen" und "denaturierenden" Bedingungen lediglich wenige Minuten benötigt. Die Auswertung der Spektren und die entsprechenden Berechnungen der Intensitäten sowie des Überschussverhältnisses kann leicht computerunterstützt vollzogen werden. Der Einsatz von Eichsubstanzen ist, bis auf einen internen Standard, der jedes Mal der gleiche sein kann, nicht notwendig. Somit kann, aufgrund der vorstehenden Vorteile, der Fehler bei der Bestimmung des Doppelstranganteils auf unter 2 Prozent gedrückt werden.

Das erfindungsgemäße Verfahren ist mit jedem MALDI-Massenspektrometer durchführbar, solange es sich um ein lineares MALDI-Massenspektrometer handelt.

Die in dem erfindungsgemäßen Verfahren verwendbaren MALDI-Probenmatrizes sind neutrale Matrizes, die nicht zu einer Denaturierung der Doppelstränge führen. Als besonders vorteilhaft haben sich gesättigte Lösungen von 6-Aza-2-thiothymin (ATT, erhältlich von Fluka, Deutschland) (ca. 6 - 7 mg/ml) in 100 - 200 mM, besonders vorteilhaft 120 - 150 mM, wässriger Diammoniumhydrogencitrat-Lösung (DAHC, erhältlich von Fluka, Deutschland), herausgestellt. Mit von 0 mM ansteigender Konzentration des DAHC nimmt die Konzentration des detektierbaren Doppelstrangs zu, und nähert sich bei 150 - 200 mM einer Sättigung (vgl. Bsp. 2). Ab 200 mM DAHC kann es zu Ausfällung gelöster Stoffe kommen. Typischerweise werden 2 μL dieser Matrixlösung mit 0,5 μL einer OligonukleotidLösung von ca. 20 μM direkt auf dem Probenteller gemischt und im kalten Luftstrom getrocknet. Erfindungsgemäß besonders wichtig ist der Verzicht auf organische Lösungsmittel, wie z.B. Acetonitril, bei der MALDI-Probenbereitung, welche sonst im Stand der Technik zur Verbesserung der Löslichkeit der Proben eingesetzt werden. Die effektive Probenkonzentration von ca. 20 μM liegt im Bereich der üblicherweise verwendeten, wobei eine Verringerung der Probenkonzentration eine abnehmende Stabilität des Doppelstrangs bewirken würde. Zur Erreichung eines ausreichenden Signal-Rausch-Verhältnisses werden typischerweise 50 - 100 Einzelspektren akkumuliert.

Besonders vorteilhaft ist das erfindungsgemäße Verfahren bei einer Länge der Nukleinsäuren von 10 bis 60 bp, insbesondere von 18 - 23 bp, z.B. von siRNA. Eine untere Grenze für eine ausreichende Stabilität des Doppelstrangs dürfte bei ca. 10 bp liegen.

Die Erfindung betrifft weiterhin die Verwendung einer Säure zur Denaturierung von Nukleinsäuren in der Probenbereitung für die Massenspektrometrie.

Weiter betrifft die Erfindung die Verwendung eines Gemisches von 6-Aza-2-thiothymin (ATT) und Diammoniumhydrogencitrat (DAHC) in einer Probenmatrix für die MALDI-Massenspektrometrie.

Das erfindungsgemäße Verfahren eignet sich auch zum Nachweis von anderen, nicht kovalenten Wechselwirkungen zwischen Nukleinsäureeinzelsträngen, die komplementäre Basenfolgen aufweisen. So kann über das Verhältnis zwischen als Einzelstrang und als Doppelstrang vorliegenden Spezies auf die Bindungsenergie zur Bildung des Doppelstrangs aus den jeweiligen Strängen rückgeschlossen werden. Entsprechende Verfahren zur Bestimmung der Bindungsenergien aus den Bindungsverhältnissen sind im Stand der Technik bekannt.

Des Weiteren kann das erfindungsgemäße Verfahren zur Bestimmung von Proteinwechselwirkungen verwendet werden, da entsprechend obiger Ausführungen auf identische Weise das Verhältnis zwischen dissoziierten Proteinen und Proteinkomplexen bestimmt werden kann. Aus dem mittels erfindungsgemäßen Verfahren bestimmten Verhältnis kann auf entsprechende Wechselwirkungsenergien rückgeschlossen werden.

Des Weiteren kann das erfindungsgemäße Verfahren zur Qualitätskontrolle synthetisch hergestellter, doppelsträngiger Oligonukleotide, wie sie z.B. in Genexpressionsstudien vorkommen, verwendet werden (siRNA bzw. RNAi).

Schließlich kann das erfindungsgemäße Verfahren vorteilhaft zur Herstellung von Mischungen mit definiertem Verhältnis zwischen Doppelstrang und Einzelstrang verwendet werden. Dies ist z.B. über Mischungstitration mit anschließender erfindungsgemäßer MALDI-Massenspektrometrie möglich. Da für jedes Titrationsstadium schnell und einfach der Einzelstrangüberschuss bestimmt werden kann, wird somit ein einfaches Verfahren zur Verfügung gestellt, um Verbindungen zu erhalten, die einen definierten Anteil von Doppelstrang, vorzugsweise hauptsächlich aus Doppelstrang, z.B. > 98%, bestehen. Derartige Verbindungen sind gerade für pharmazeutische Applikationen von großer Bedeutung.

Figur 1 zeigt eine Grafik mit zwei versetzt dargestellten Massenspektren einer Probe bei nativen und denaturierenden Bedingungen.

Figur 2 zeigt ein Diagramm, in dem die Doppelstrang/Einzelstrang-Intensität einer nukleinsäurehaltigen Probe gegen die DAHC-Konzentration der Probenlösung aufgetragen ist.

Die Erfindung wird anhand folgender Beispiele weiter veranschaulicht:

**Beispiel 1**

Quantifizierung des Doppelstranganteils

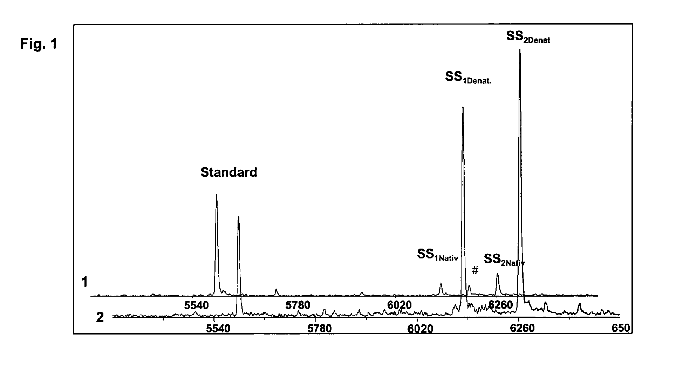

Figur 1 zeigt zwei versetzt dargestellte MALDI-MS-Spektren, wobei Spur 1 unter "nativen" Bedingungen und Spur 2 unter "denaturierenden" Bedingungen aufgenommen wurde. Es ist nur der Massenbereich des Standards und der Einzelstränge gezeigt, da die Peaks des Doppelstrangs für die Auswertung nicht benötigt werden. Als Standard wurde ein Oligonukleotidstrang eingesetzt, der mit den zu untersuchenden komplementären Einzelsträngen 1 und 2 ($SS_1$ und $SS_2$) nicht interagiert. Da der Standard in den Proben der beiden Messungen identisch ist, können über den Korrekturfaktor y, der gemäß obiger Formel (1) bestimmt wird, die Intensitäten der beiden Spektren normalisiert werden, so dass anschließend die Intensität des Standards in beiden Spektren gleich ist und die jeweiligen Einzelstrangintensitäten somit direkt vergleichbar werden. Für das in Figur 1 gezeigte Spektrum wurde nach der Normalisierung auf den Standard für Einzelstrang 1 ($SS_1$) eine Intensität von 756 counts unter nativen und 13227 counts unter denaturierten Bedingungen gemessen. Es ergibt sich nach obiger Formel (3) ein Doppelstranganteil bezogen auf den Einzelstrang 1 von 100 - 756/13227 x 100 = 94,3%.

Des Weiteren kann ein eventueller Überschuss eines Einzelstrangs bestimmt werden. Neben den bekannten Werten für Einzelstrang 1 ($SS_1$) werden noch die Werte des Einzelstrangs 2 ($SS_2$) der Peaks bei "nativen" Bedingungen (1347 counts) und bei "denaturierenden" Bedingungen (16749 counts) benötigt. In die oben angegebene Formel (4b) eingesetzt ergibt sich somit ein relativer Überschuss für Einzelstrang 2 von 2,5 %. Das heißt, Einzelstrang 1 liegt insgesamt 5,7% in Form des Einzelstrangs und Einzelstrang 2 8,2% in Form des Einzelstrangs vor, wobei Einzelstrang 2 einen relativen Überschuss von 2,5 % aufweist.

Bei den Untersuchungen wurde als Probenmatrix eine gesättigte Lösung von 6-Aza-2-thiothymin (ATT) in 100 - 200 mM wässriger Diammoniumhydrogencitrat-Lösung (DAHC) verwendet. 2 μL dieser Matrixlösung wurden mit 0,5 μL Oligonukleotidlösung (ca. 20 μM) direkt auf dem Probenteller gemischt und in einem kalten Luftstrom getrocknet.

Probensequenz (RNA): 5' G*GC*GA*UA*UU*CU*GC*UA*CA*GU*

x ACUGUAGCAGAAUAUCGCC 3'

(* = 2' Omethyl)

Als Standard wurde ein RNA-Oligonukleotid mit der Länge von 17 Basenpaaren eingesetzt, der unter den experimentellen Bedingungen nicht mit der Probe interagierte. Die massenspektrometrische Analyse wurde mit einem MALDI-Flugzeitmassenspektrometer (Voyager De-Pro, Applied Biosystems, Framingham, USA) im Linearbetrieb durchgeführt. Es können Spektren der positiven Ionen als auch der negativen Ionen zur Analyse verwendet werden, da beide vergleichbare Intensitäten und Auflösungen zeigen. Als Betriebsparameter wurden eine Beschleunigungsspannung von 20 kV, eine Grid-Spannung von 95% der Beschleunigungsspannung und eine Delay-Zeit von 600 ns gewählt. Um ein ausreichendes Signal-Rausch-Verhältnis zu erhalten, wurden für jedes Spektrum 50 - 100 Einzelspektren akkumuliert. Zur Berechnung der Peakflächen bzw. -höhen wurden die Spektren 19 pt geglättet und die Basislinie korrigiert.

**Beispiel 2**

Einzelstrang/Doppelstrangverhältnis bei verschiedenen DAHC-Konzentrationen

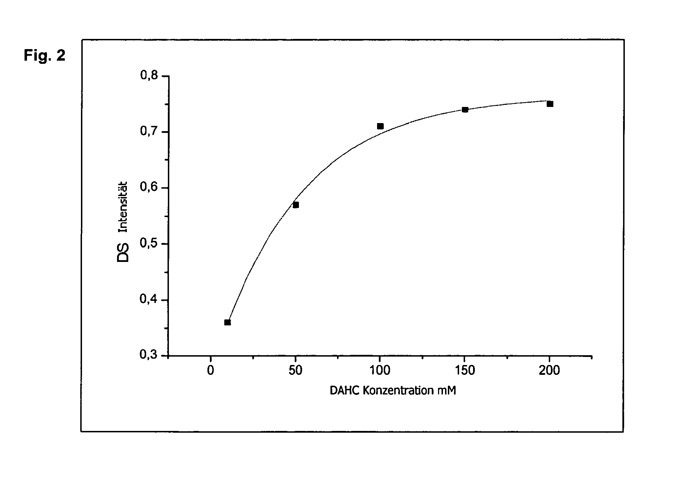

In Figur 2 wurde das Verhältnis von Einzelstrang zu Doppelstrang für verschiedene Konzentrationen von DAHC untersucht. Die angegebenen Werte für die Doppelstrangintensität sind willkürliche Einheiten und entsprechen nicht den wirklichen Verhältnissen.

Es wurde gefunden, dass die Konzentration an Doppelstrang mit zunehmender DAHC-Konzentration zunimmt, sie nähert sich bei 150 - 200 mM einer Sättigung.

Die Proben wurden gemäß Beispiel 1 bereitet und vermessen. Die Verhältnisse von Doppelstrang zu Einzelstrang wurden gemäß dem oben angegebenen Verfahren der gemäß Beispiel 1 gemessenen Spektren berechnet.

**Patentansprüche**

1.  Verfahren zur Bestimmung wenigstens eines Eigenschaftsparameters einer Probe, die wenigstens ein Biomolekül enthält, wobei das Verfahren die folgenden Schritte umfasst:

    (a) Zugabe eines Standards zu der Probe;
    (b) Erstellen eines Massenspektrums der den Standard enthaltenden Probe unter nativen Bedingungen;
    (c) Erstellen eines Massenspektrums der den Standard enthaltenden Probe unter denaturierenden Bedingungen;
    (d) Vergleichen der Peakhöhe oder Peakfläche von wenigstens einem dem Biomolekül zuzuordnenden Peak in dem Massenspektrum aus Schritt (b) mit der Peakhöhe bzw. Peakfläche des korrespondierenden Peaks in dem Massenspektrum aus Schritt (c).

2.  Verfahren gemäß Anspruch 1, **dadurch gekennzeichnet, dass** das Biomolekül mindestens eine Nukleinsäure umfasst.

3.  Verfahren gemäß einem der Ansprüche 1 oder 2, **dadurch gekennzeichnet, dass** das Verhältnis der dem Standard entsprechenden Peaksignale in den Massenspektren aus den Schritten (b) bzw. (c) zueinander berücksichtigt wird.

4.  Verfahren gemäß einem der Ansprüche 1-3, **dadurch gekennzeichnet, dass** der Eigenschaftsparameter ausgewählt ist aus der Gruppe bestehend aus Doppelstranganteil der Probe, Verhältnis von Einzelsträngen zu Doppelsträngen in der Probe, Überschuss an einem Einzelstrang bezüglich eines zweiten Einzelstrangs in der Probe, Wechselwirkungskräfte zwischen verschiedenen Nukleinsäuren der Probe und Wechselwirkung von in der Probe vorhandenen Nukleinsäuren mit anderen nichtnukleidischen Inhaltsstoffen der Probe.

5.  Verfahren gemäß Anspruch 4, **dadurch gekennzeichnet, dass** der Eigenschaftsparameter der Doppelstranganteil der Probe, das Verhältnis von Einzelsträngen zu Doppelsträngen der Probe und/oder das Verhältnis von Einzelstrang zu Doppelstrang in der Probe ist.

6.  Verfahren gemäß einem der Ansprüche 1 - 5, **dadurch gekennzeichnet, dass** die denaturierenden Bedingungen durch Zugabe einer Säure hergestellt werden.

7.  Verfahren gemäß einem der Ansprüche 1 - 6, **dadurch gekennzeichnet, dass** das Massenspektrometer auf MALDI basiert.

8.  Verfahren gemäß Anspruch 7, **dadurch gekennzeichnet, dass** die MALDI-Probenmatrix 6-Aza-2-thiothymin (ATT) und Diammoniumhydrogencitrat (DAHC) umfasst.

**9.** Verfahren gemäß einem der vorigen Ansprüche, **dadurch gekennzeichnet, dass** der Standard eine einzelsträngige Nukleinsäure ist.

**10.** Verwendung von Säure zur Denaturierung von Nukleinsäuren in der Probenbereitung für die Massenspektrometrie.

**11.** Verwendung eines Gemisches von 6-Aza-2-thiothymin (ATT) und Diammoniumhydrogencitrat (DAHC) in einer Probenmatrix für die MALDI-Massenspektrometrie.

**Fig. 1**

**Fig. 2**

**Europäisches Patentamt**

# EUROPÄISCHER RECHERCHENBERICHT

**Nummer der Anmeldung**

EP 06 02 5448

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 99/57318 A (SEQUENOM INC [US]; HILLENKAMP FRANZ [DE]; KOESTER HUBERT [US]) 11. November 1999 (1999-11-11) * Beispiel 3 * | 1-7,9,10 | INV. C12Q1/68 G01N33/68 |
| X | SIMMONS T A ET AL: "Influence of Co-matrix Proton Affinity on Oligonucleotide Ion Stability in Matrix-Assisted Laser Desorption/Ionization Time-of-Flight Mass Spectrometry" JOURNAL OF THE AMERICAN SOCIETY FOR MASS SPECTROMETRY, ELSEVIER SCIENCE INC, US, Bd. 9, Nr. 7, Juli 1998 (1998-07), Seiten 668-675, XP004133304 ISSN: 1044-0305 | 11 | |
| A | * section "Sample preparation" * * Seite 673, Spalte 1, Zeile 10 - Seite 674, Spalte 2, Zeile 10; Abbildungen 4,5 * | 7-9 | |
| X | DISTLER A M ET AL: "5-methoxysalicylic acid and spermine: a new matrix for the matrix-assisted laser desorption/ionization mass spectrometry analysis of oligonucleotides" JOURNAL OF THE AMERICAN SOCIETY FOR MASS SPECTROMETRY, ELSEVIER SCIENCE INC., NEW YORK, NY, US, Bd. 12, Nr. 4, April 2001 (2001-04), Seiten 456-462, XP004233063 ISSN: 1044-0305 *"Results and Discussion", Abs.1-2* Abbildung 2 * | 11 | RECHERCHIERTE SACHGEBIETE (IPC) C12Q G01N |
| X | WO 2004/031730 A (ANDERSON NORMAN LEIGH [US]) 15. April 2004 (2004-04-15) * Anspruch 36 * | 1,3 | |

-/--

Der vorliegende Recherchenbericht wurde für alle Patentansprüche erstellt

| Recherchenort | Abschlußdatum der Recherche | Prüfer |
|---|---|---|
| München | 29. Mai 2007 | Luzzatto, Enrico |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches
Patentamt

**EUROPÄISCHER RECHERCHENBERICHT**

Nummer der Anmeldung

EP 06 02 5448

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2006/015797 A (HAFTEN E V MAX PLANCK GES ZUR [DE]; SAUER SASCHA [DE]; BUESSOW KONRAD) 16. Februar 2006 (2006-02-16) * Seite 13, Zeile 10 - Seite 17, Zeile 9 * * Seite 23, Absatz 1 - Absatz 3; Beispiel 1 * ----- | 1-3,6,7, 10 | |
| A | BENNER WILLIAM H ET AL: "Identification of denatured double-stranded DNA by matrix-assisted laser desorption/ionization time-of-flight mass spectrometry" RAPID COMMUNICATIONS IN MASS SPECTROMETRY, Bd. 9, Nr. 6, 1995, Seiten 537-540, XP009084392 ISSN: 0951-4198 * das ganze Dokument * ----- | 1,2 | |
| A | LECCHI ET AL: "The detection of intact double-stranded DNA by MALDI" JOURNAL OF THE AMERICAN SOCIETY FOR MASS SPECTROMETRY, ELSEVIER SCIENCE INC, US, Bd. 6, Nr. 10, Oktober 1995 (1995-10), Seiten 972-975, XP005271103 ISSN: 1044-0305 * das ganze Dokument * ----- | 1,2 | RECHERCHIERTE SACHGEBIETE (IPC) |
| A | BARTOLINI W P ET AL: "Identification of single stranded regions of DNA by enzymatic digestion with matrix-assisted laser desorption/ionization analysis - A Laboratory Manual" JOURNAL OF THE AMERICAN SOCIETY FOR MASS SPECTROMETRY, ELSEVIER SCIENCE INC., NEW YORK, NY, US, Bd. 10, Nr. 6, Juni 1999 (1999-06), Seiten 521-528, XP004173042 ISSN: 1044-0305 * das ganze Dokument * ----- | 2-7 | |

Der vorliegende Recherchenbericht wurde für alle Patentansprüche erstellt

| Recherchenort | Abschlußdatum der Recherche | Prüfer |
|---|---|---|
| München | 29. Mai 2007 | Luzzatto, Enrico |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
...............................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 06 02 5448

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-05-2007

| Im Recherchenbericht angeführtes Patentdokument | Datum der Veröffentlichung | Mitglied(er) der Patentfamilie | Datum der Veröffentlichung |
|---|---|---|---|
| WO 9957318 A | 11-11-1999 | AU 3896799 A | 23-11-1999 |
| | | CA 2328110 A1 | 11-11-1999 |
| | | DE 19983215 T0 | 17-05-2001 |
| | | EP 1075545 A2 | 14-02-2001 |
| | | JP 2002513917 T | 14-05-2002 |
| | | NO 20005539 A | 05-01-2001 |
| | | US 2001055811 A1 | 27-12-2001 |
| | | US 6558902 B1 | 06-05-2003 |
| | | US 2003148528 A1 | 07-08-2003 |
| WO 2004031730 A | 15-04-2004 | AU 2003287017 A1 | 23-04-2004 |
| | | CA 2501000 A1 | 15-04-2004 |
| | | EP 1556478 A2 | 27-07-2005 |
| WO 2006015797 A | 16-02-2006 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur

- **ELBASHIR SM ; LENDECKEL W ; TUSCHL T.** *Genes Dev,* 2001, vol. 15 (2), 188-200
- **ELBASHIR SM ; HARBORTH J ; LENDECKEL W ; YALCIN A ; WEBER K ; TUSCHL T.** *Nature,* 2001, vol. 411, 494-498
- **CAPLEN NJ ; PARRISH S ; IMANI F ; FIRE A ; MORGAN RA.** *Proc. Natl. Acad. Sci. USA,* 2001, vol. 98, 9746-9747
- **HEIL F ; HEMMI H ; HOCHREIN H ; AMPENBERGER F ; KIRSCHNING C ; AKIRA S ; LIPFORD G ; WAGNER H ; BAUER S.** *Science,* 2004, vol. 303, 1526-1529
- **W. D. LEHMANN.** Massenspektrometrie in der Biochemie. Spektrum Akad. Verlag, 1996
- **M. KARAS ; F. HILLENKAMP.** *Anal. Chem.,* 1988, vol. 60, 2299
- **M. KARAS ; U. BAHR ; F. HILLENKAMP.** *Int. J. Mass Spectrom. Ion Proc.,* 1989, vol. 92, 231
- **M. KARAS ; U. BAHR ; A. LNGENDOH ; F. HILLENKAMP.** *Angew. Chemie,* 1989, vol. 101, 805
- **F. HILLENKAMP ; M. KARAS.** *Methods Enzymol.,* 1990, vol. 193, 280
- **BONK T ; HUMENY A.** *The Neuroscientist,* 2001, vol. 7 (1), 6-12
- **DISTLER AM ; ALLISON J.** *Anal Chem.,* 2001, vol. 73 (20), 5000-5003
- **LECCI ; PANNELL.** *J. Am. Soc. Mass Spectrom.,* 1995, vol. 6, 972-975
- **LITTLE et al.** *Int. J. Mass Spectrom. Ion Processes,* 1997, vol. 169/170, 323-330
- **KIRPEKAR F ; BERKENKAMP S ; HILLENKAMP F.** *Anal. Chem.,* 1999, vol. 71 (13), 2334-2339